# FASCICULE DE BREVET EUROPEEN

(11) **EP 0 610 413 B1**
(45) Date de publication et mention de la délivrance du brevet: **22.05.1996**
(21) Numéro de dépôt: 92923859.0
(22) Date de dépôt: 26.10.1992
(51) Int. Cl.: A61M 16/04

(54) **NEZ ARTIFICIEL POUR SUJET TRACHEOTOMISE**
KUNSTLICHE MASE FUR JEMANDEN MIT EINER TRACHEOTOMIE
ARTIFICIAL NOSE FOR A TRACHEOTOMY PATIENT

(30) Priorité: 30.10.1991 FR 9113378
(43) Date de publication de la demande: 17.08.1994
(73) Titulaire: BEZICOT, Robert, F-92160 Antony (FR)
(72) Inventeur: BEZICOT, Robert, F-92160 Antony (FR)
(74) Mandataire: CABINET BONNET-THIRION
(86) Numéro de dépôt international: FR9200999
(87) Numéro de publication internationale: WO9308860

(56) Documents cités:
- WO-A-91/05579
- DE-A- 3 606 137
- US-A- 2 491 647

## Description

L'invention se rapporte à un nez artificiel pour un sujet trachéotomisé avec un orifice chirurgical abouchant la trachée à la peau, comportant un boîtier ajouré tenu à la périphérie de l'orifice chirurgical par une embase annulaire adhérant à la peau, une masse filtrante hydrophile formant échangeur à circulation alternée de chaleur et d'humidité entre l'air exhalé et l'air inhalé, une grille étant disposée entre la masse filtrante et l'orifice chirurgical.

Etant donné que, chez les sujets trachéotomisés, l'air inspiré débouche directement dans la trachée, il est très important, pour le confort respiratoire et la santé du sujet que cet air inspiré soit sensiblement à la même température, et aux mêmes teneurs en humidité et poussières que s'il parvenait à la trachée après avoir traversé les voies respiratoires supérieures, narines, fosses nasales, pharynx et larynx, c'est-à-dire à une température approchant 32°C, avec une teneur en humidité proche de la saturation à la température de cet air, et débarrassé de la majorité des poussières.

Les dispositions énoncées ci-dessus permettent d'approcher un tel résultat ; la masse filtrante arrête bien entendu une partie importante des poussières en suspension dans l'air ; la vapeur d'eau contenue dans l'air exhalé, qui est à saturation à la température de l'organisme, se condense sur la masse filtrante, qui se trouve ainsi réchauffée sensiblement à la température du corps ; l'air inhalé, arrivant à la température de l'air ambiant, se réchauffe et se charge en humidité au contact de la masse filtrante à température supérieure et contenant l'eau condensée.

Toutefois ces dispositions connues, notamment par le document WO-A-91/05579, ne procurent pas une protection suffisante contre un colmatage des pores de la masse filtrante par des mucosités expulsées de la trachée. Ces mucosités, dues à la défense de l'organisme contre des corps étrangers tels que poussières, micro-organismes et cellules mortes, devraient être arrêtées par la grille située entre l'orifice chirurgical et la masse filtrante ; toutefois le courant d'air à l'expiration a tendance à entraîner les mucosités de la face postérieure où elles se sont déposées vers la face antérieure, et de-là les étaler sur la surface de la masse filtrante au contact de la face antérieure de la grille. La dessication subséquente des mucosités conduit à la formation de croûtes durcies, qui colmatent la masse filtrante. Le nez artificiel doit alors être remplacé.

L'invention a pour objectif essentiel un nez artificiel amélioré, pour lequel le colmatage de la masse filtrante est fortement retardé, sinon évité.

A cet effet, l'invention propose un nez artificiel pour un sujet trachéotomisé avec un orifice chirurgical abouchant la trachée à la peau, comportant dans un boîtier ajouré tenu à la périphérie de l'orifice chirurgical par une embase annulaire adhérant à la peau, une masse filtrante hydrophile formant échangeur à circulation alternée de chaleur et d'humidité entre l'air exhalé et l'air inhalé, une grille étant disposée entre la masse filtrante et l'orifice chirurgical, caractérisé en ce que, la grille étant disposée dans le boîtier avec des surfaces antérieure et postérieure distantes dans leur partie centrale active et tournées respectivement vers la masse filtrante et l'orifice chirurgical, la surface postérieure possède des passages calibrés, et est espacée de la surface antérieure d'une distance telle, conjointement avec le calibre des passages, que des mucosités expulsées de la trachée ne puissent, pour l'essentiel, atteindre la masse filtrante.

Le Demandeur a découvert que les propriétés rhéologiques des mucosités, viscosité et tension superficielle, étaient telles que, après s'être collées à une grille d'ouverture appropriée à les arrêter, ces mucosités étaient susceptibles de s'allonger dans le sens du courant d'air expiré sans se détacher ni se rompre, et sur une longueur notable, fonction des dimensions de passage de la grille ; en conséquence, en utilisant une grille comportant deux surfaces, antérieure au contact de la masse filtrante, et postérieure exposée à l'impact des mucosités expulsées de la trachée, et en écartant ces grilles d'une distance supérieure à l'allongement possible des mucosités, on évite que les mucosités arrêtées par la surface extérieure n'atteignent la masse filtrante pour la colmater.

Le document US-A-2 491 647 décrit un dispositif de nez artificiel qui comporte une grille filtre pour éviter que les mucosités expulsées par le sujet en toussant ne soient projetées dans l'environnement. Mais ce nez artificiel ne comporte pas de masse filtrante pour réchauffer et humidifier l'air aspiré.

De préférence, la masse filtrante est réalisée en mousse de polyuréthanne à pores ouverts, cette matière associant à un caractère hydrophile convenable des facilités de mise en forme et de durabilité appropriées, un prix modique et des facilités de nettoyage intéressantes.

De préférence, l'embase comporte une collerette souple entourant un rebord annulaire antérieur sur lequel s'engage un alésage complémentaire du boîtier. Cette disposition permet un accès facile à la masse filtrante et à la grille, après avoir détaché le nez artificiel de l'entourage de l'orifice chirurgical, pour le remplacement de la masse filtrante et le nettoyage de la grille.

En outre, le rebord périphérique de l'embase peut comporter un jonc périphérique qui s'engage dans une gorge complémentaire pratiquée dans l'alésage du boîtier.

Il est préférable, pour assurer la coïncidence du nez artificiel et de l'orifice chirurgical, que l'embase comporte en outre une manchette postérieure, dans le prolongement du rebord annulaire et apte à s'engager dans l'orifice chirurgical. On obtient ainsi un centrage exact de l'alésage du boîtier sur l'orifice chirurgical, tout en protégeant la paroi de cet orifice contre un contact intempestif de la surface postérieure de grille.

En disposition préférée, l'embase est garnie d'un adhésif double face, susceptible d'être renouvelé pour fixer efficacement l'embase sur la peau sans avoir à remplacer l'embase en son entier.

De préférence, la surface antérieure de grille est plane, tandis que sa forme postérieure est bombée en dôme et constituée d'une grille à mailles carrées. Cette forme permet une réalisation aisée et robuste.

Selon une disposition préférée, le boîtier, de forme annulaire avec un fond antérieur grillagé, comporte un capot recouvrant le fond antérieur parallèlement et à distance de celui-ci, avec des ouvertures latérales.

Ainsi le boîtier en soi est de forme simple, facile à nettoyer, et constitue, par son fond antérieur grillagé, une surface d'entrée d'air de dimensions relativement grande, permettant notamment la pulvérisation d'aérosols de médication. Mais le capot vient recouvrir ce fond antérieur, ce qui permet notamment d'éviter qu'un col un peu haut ne vienne couvrir hermétiquement le fond grillagé.

En outre, le capot peut comporter un bord latéral qui s'étend jusqu'au fond antérieur sur une demi-circonférence, tandis que l'autre demi-circonférence débouche dans une jupe plate orientable. Cette disposition permet, en orientant la jupe verticalement vers le bas, de prendre une douche sans que le fond antérieur du boîtier soit noyé, le capot formant parapluie.

Le capot peut également comporter, coiffant le boîtier autour du fond antérieur, une chambre à clapets, avec un clapet d'aspiration entre la chambre et une canalisation apte à être plaquée sur le thorax du sujet en sorte d'être sensiblement à la température du corps, et un clapet d'expiration débouchant directement à l'extérieur.

On obtient ainsi un réchauffement extrêmement efficace de l'air inspiré, sans que pour autant le prolongement du chemin d'air inhalé soit occupé par de l'air exhalé, chargé en gaz carbonique et autres pollutions gazeuses expirées.

Des caractéristiques secondaires et avantages de l'invention ressortiront d'ailleurs de la description qui va suivre à titre d'exemple, en référence aux dessins annexés dans lesquels :
la figure 1 est une vue latérale coupée d'un nez artificiel selon l'invention ;
la figure 2 est une vue de face d'un nez artificiel muni d'un capot passe-partout ;
la figure 3 est une vue latérale d'un nez artificiel muni d'un capot passe-partout ;
les figures 4A et 4B sont des vues, respectivement de face et de côté, d'un nez artificiel muni d'un capot de protection contre des ruissellements de liquide ;
la figure 5 est une vue en coupe transversale d'un nez artificiel muni d'un capot prévu pour assurer un réchauffage amélioré de l'air inspiré.

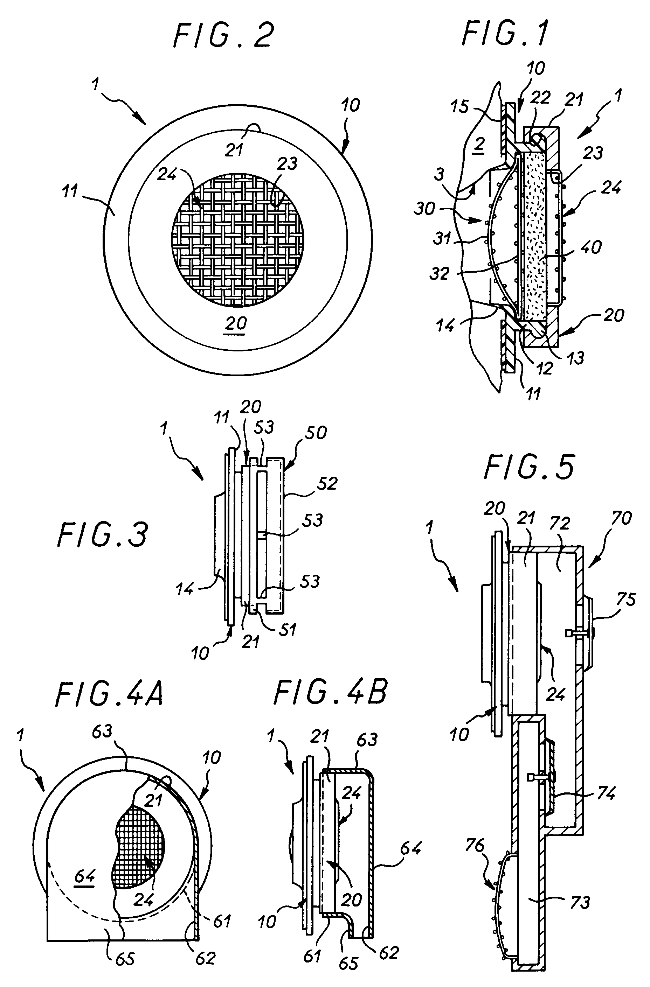

Selon la forme de réalisation choisie et représentée aux figures 1 et 2, le nez artificiel 1 dans son ensemble est composé d'une embase en élastomère 10, circulaire, et d'un boîtier 20, en matière rigide, métal ou polymère moulé. L'embase 10 comporte une collerette en couronne 11, autour d'un rebord annulaire 12. La collerette 11 est fixée à la peau de la base du cou 2 d'un sujet trachéotomisé, autour d'un orifice chirurgical 3 qui abouche la trachée à la peau, par une bande adhésive double face 15, qui assure une liaison étanche. Une manchette 14, à peu près dans le prolongement du rebord 12, fait saillie autour de l'évidement central de la collerette 11 pour s'engager dans l'orifice chirurgical 3, et assurer le centrage du nez artificiel 1 par rapport à cet orifice chirurgical.

Si l'on prend pour référence d'orientation le sujet trachéotomisé, le rebord 12 occupe une position antérieure et la manchette 14 une position postérieure, par rapport à la collerette 11. Dans ce qui suit, ces termes "antérieur" et "postérieur" seront utilisés par référence d'orientation au sujet porteur du nez artificiel.

Le rebord 12 est constitué d'un tronçon de tube ; à son extrémité antérieure, il est muni d'un jonc 13 en saillie de la périphérie du tronçon de tube.

Le boîtier 20, en forme générale de disque, comporte un bord tubulaire 21, dans l'alésage duquel est ménagée une gorge 22 complémentaire du jonc 13 de façon à fixer, de façon amovible, le boîtier 20 sur l'embase 10. La face antérieure du boîtier 20 est ajourée par une fenêtre circulaire 23, qui se place dans le prolongement de l'évidement central de l'embase 10, cette fenêtre étant munie d'un grillage 24.

Dans le boîtier 20 est logée une masse filtrante 40, en forme de disque et constituée d'une mousse de polyuréthanne à pores ouverts, à propriétés hydrophiles.

Entre cette masse filtrante 40 et l'orifice chirurgical 3 est disposée une grille d'arrêt 30 dans son ensemble. Cette grille 30 comporte une surface postérieure bombée 31, et une surface antérieure plane 32, ces surfaces étant réunies par leurs bords circulaires en une couronne qui bute sur la périphérie antérieure de l'évidement central de l'embase 10. La surface postérieure bombée 31 et ici la surface antérieure 32 sont constituées d'un grillage à maille carrée à pas de 4 mm environ.

On notera que la manchette 14, outre qu'elle facilite le centrage du nez artificiel dans l'orifice chirurgical, vient empêcher que la surface postérieure 31 de la grille 30 vienne porter directement sur le bord de l'orifice chirurgical 3, malgré que son bombement l'amène à pénétrer dans cet orifice.

De façon connue, le disque filtrant 40, qui se trouve refroidi par l'air ambiant, va provoquer la condensation d'une partie de l'humidité contenue dans l'air exhalé, humidité qui atteint la saturation à la température des poumons. L'humidité condensée reste pour l'essentiel dans la masse filtrante, en raison de ses propriétés hydrophiles. La condensation produit une élévation de température de la masse filtrante, par dégagement de la chaleur latente de vaporisation. Lors de l'inhalation subséquente, l'air inhalé se chargera en humidité par évaporation d'une partie de l'humidité retenue par la masse filtrante, tout en se réchauffant au contact de l'humidité qui reste retenue. Après quelques cycles de respiration, exhalation et inhalation, un état de régime s'établit, dans des conditions analogues à ce qui se passe lors de la respiration naturelle par les voies respiratoires supérieures.

La fonction de la grille d'arrêt conforme à l'invention est d'arrêter les mucosités expulsées de la trachée. Ces mucosités, formées par réaction organique de défense contre les agressions par les poussières, micro-organismes et cellules mortes détachées notamment, sont constituées de gels aqueux à viscosité souvent élevée, et tension superficielle suffisante pour leur conférer la consistance de globules autonomes.

Lorsque ces mucosités parviennent au contact de la masse filtrante humide 40, elles ont tendance à s'y étaler, puis à se dessécher au contact de l'air inhalé auquel elles cèdent l'eau de constitution du gel. Il se produit alors un colmatage de la masse filtrante.

Mais, les mucosités de relativement gros volume, qui seraient responsables pour l'essentiel du colmatage de la masse filtrante viennent frapper la surface postérieure bombée 31 de la grille 30 et s'y attachent. Leurs viscosités et tensions superficielles empêchent qu'elles se fragmentent par entraînement par l'air exhalé, de sorte qu'elles s'allongent suivant ce courant d'air tout en restant adhérentes à la surface postérieure de la grille, dans l'espace situé entre la surface postérieure 31 et la surface antérieure 32, sans atteindre la masse filtrante 40.

On comprend que, si la maille de la surface postérieure 31 de grille 30 est trop faible, les mucosités viendront colmater la grille. Par contre, si la maille est trop grande, la probabilité d'arrêt des mucosités par la face postérieure 31 de la grille 30 devient faible, et la grille perd de son efficacité. On a déterminé expérimentalement qu'une maille carrée de 4 mm, avec un diamètre de fil voisin du millimètre, convenait bien.

On aura compris que la face antérieure plane 32 de la grille 30 a seulement pour but de maintenir en place la masse filtrante 40, pour qu'elle ne soit pas avalée à l'inspiration jusqu'au contact de la surface postérieure 31.

La présence de l'adhésif double face 15, physiologiquement acceptable bien sûr, permet la réutilisation du nez artificiel après qu'il ait été enlevé, en remplaçant précisément la couronne d'adhésif double face 15 qui a perdu ses propriétés adhésives après avoir été détaché. L'ouverture facile du boîtier permet, en outre, le remplacement de la masse filtrante 40 usagée, et le nettoyage de la grille 30.

On remarquera que la dimension relativement importante de la fenêtre 23 permet l'usage d'aérosols à fin de médication, ces aérosols étant projetés sur la masse filtrante 40 à travers la grille 24.

Mais, dans la vie courante, la fenêtre 23 ouverte frontalement présente l'inconvénient d'être exposée directement à des contacts, notamment de vêtements, et n'est pas précisément esthétique.

La figure 3 représente, à échelle plus petite, un nez artificiel 1, qui est muni d'un capot 50, prévu principalement pour éviter que la fenêtre 23 du couvercle 20 soit obturée par des vêtements. Ce capot 50 se compose d'un anneau 51 qui se glisse à frottement sur le bord 21 du boîtier 20, d'une plaque frontale 52, de même diamètre extérieur que l'anneau 51, et d'entretoises 53 entre l'anneau et la plaque frontale 52. Outre sa fonction de protection de la fenêtre 23 du boîtier, ce capot 50 masque l'ouverture grillagée et peut porter un décor qui atténue l'aspect fonctionnel du boîtier.

En outre le capot 50 selon la figure 3 est utilisable par les sujets qui portent un implant dit sonatoire pour rétablir la parole par la bouche, le fonctionnement de cet implant nécessitant que soit obturé temporairement l'orifice chirurgical. Dans ce but, la course de coulissement de l'anneau 51 sur la périphérie ou bord 21 de boîtier 20 est suffisante pour que la plaque frontale 52, normalement distante de la fenêtre 23, puisse être enfoncée jusqu'à venir au contact du fond antérieur du boîtier et obturer la fenêtre 23.

Les figures 4A et 4B représentent un capot prévu plus particulièrement pour mettre le nez artificiel 1 à l'abri de ruissellement de liquide, lors d'une douche, par exemple, ou lors d'activités nautiques. Le capot 60 vient s'insérer par un anneau circulaire 61 sur le bord périphérique 21 du boîtier de nez artificiel 1, à frottement. Sur une demi-circonférence, l'anneau 61 est prolongé par un bord 63 qui s'étend jusqu'à une plaque frontale 64. Sur l'autre demi-circonférence, l'anneau 61 se raccorde à une plaque 65 parallèle à la plaque frontale 64, ces plaques 64 et 65 se rejoignant latéralement pour former conjointement une jupe plate 62.

On comprend que, en dirigeant vers le bas l'ouverture de la jupe plate 62, on obtient une protection en parapluie du nez artificiel 1, qui permet de prendre une douche sans risque de noyade.

Le capot représenté figure 5 a été conçu dans le souci d'améliorer le réchauffage de l'air inhalé, notamment par temps froid. Comme on le voit, ce capot 70 dans son ensemble se compose de deux chambres oblongues, une chambre 72 qui comporte un bord en anneau 71 qui s'insère à frottement sur le bord périphérique 21 du couvercle de nez artificiel 1, et une chambre 73 située en dessous de l'anneau 71, les chambres 72 et 73 possédant une paroi commune pour permettre le recouvrement partiel des chambres. La chambre 72 possède un clapet 75 en face de la grille 24 du couvercle de nez artificiel, monté en sorte de s'ouvrir de l'intérieur vers l'extérieur en réponse à une surpression d'expiration, et un clapet 74 fermant une ouverture dans la cloison commune aux chambres 72 et 73, et monté en sorte de s'ouvrir de la chambre 73 dans la chambre 72 en réponse à une dépression d'inspiration dans la chambre 72.

La chambre 73 possède une fonction de canalisation et présente, à sa partie inférieure et dans sa paroi proche du corps du sujet, une fenêtre protégée par une grille 76 bombée, qui viendra prendre appui sur le thorax du sujet, en dessous de l'orifice chirurgical.

L'air inspiré sera ainsi puisé au contact du thorax, sous les vêtements, pour traverser la chambre 73 dont les parois sont également sensiblement à la température du corps, puis, après franchissement du clapet 74, la chambre 72 avant de parvenir au nez artificiel 1. Par contre, l'air expiré sera évacué directement de la chambre 72 vers l'extérieur par le clapet 75. On évite ainsi qu'il se forme dans la canalisation 73 qui va chercher l'air tiédi au contact du corps un volume mort d'air vicié d'expiration, chargé en gaz carbonique.

Bien entendu l'invention n'est pas limitée aux exemples décrits, mais en embrasse toutes les variantes d'exécution, dans le cadre des revendications.

On notera que la disposition selon la figure 5 pourrait être adaptée à la respiration à partir d'une alimentation par conduite en air de respiration, par exemple en plongée sous-marine avec tuba ou bouteille.

## Revendications

1. Nez artificiel (1) pour un sujet trachéotomisé avec un orifice chirurgical (3) abouchant la trachée à la peau (2), comportant dans un boîtier (20) ajouré tenu à la périphérie de l'orifice chirurgical (3) par une embase (10) annulaire adhérant à la peau, une masse filtrante hydrophile (40) formant échangeur à circulation alternée de chaleur et d'humidité entre l'air exhalé et l'air inhalé, une grille (30) étant disposée entre la masse filtrante (40) et l'orifice chirurgical (3), caractérisé en ce que, la grille (30) étant disposée dans le boîtier avec des surfaces antérieure (32) et postérieure (31) distantes dans leur partie centrale active et tournées respectivement vers la masse filtrante (40) et l'orifice chirurgical (3), la surface postérieure (31) possède des passages calibrés, et est espacée de la surface antérieure (32) d'une distance telle, conjointement avec le calibre des passages, que des mucosités expulsées de la trachée ne puissent, pour l'essentiel, atteindre la masse filtrante (40).

2. Nez artificiel selon la revendication 1, caractérisé en ce que la masse filtrante (40) est réalisée en mousse de polyuréthanne à pores ouverts.

3. Nez artificiel selon l'une des revendications 1 et 2, caractérisé en ce que l'embase (10) comporte une collerette (11) souple entourant un rebord annulaire (12) antérieur sur lequel s'engage un alésage (21) complémentaire du boîtier (20).

4. Nez artificiel selon la revendication 3, caractérisé en ce que le rebord annulaire (12) comporte un jonc périphérique (13), qui s'engage dans une gorge (22) pratiquée dans l'alésage du boîtier (20).

5. Nez artificiel selon l'une des revendications 3 et 4, caractérisé en ce que l'embase (10) comporte en outre une manchette (14) postérieure dans le prolongement du rebord annulaire (12), et apte à s'engager dans l'orifice chirurgical.

6. Nez artificiel selon l'une quelconque des revendications 1 à 5, caractérisé en ce que l'embase (10) est garnie d'un adhésif double face (15), apte à s'appliquer sur la peau (2).

7. Nez artificiel selon une quelconque des revendications 1 à 6, caractérisé en ce que la surface antérieure (32) de grille (30) est plane, tandis que sa surface postérieure (31) est bombée en dôme, et constituée d'une grille à maille carrée.

8. Nez artificiel selon une quelconque des revendications 1 à 7, caractérisé en ce que le boîtier (20) est de forme annulaire avec un fond antérieur grillagé.

9. Nez artificiel selon la revendication 8, caractérisé en ce que le boîtier (20) comporte un capot (50, 60, 70) recouvrant le fond antérieur (52, 64) parallèlement et à distance de celui-ci, avec des ouvertures latérales.

10. Nez artificiel selon la revendication 9, caractérisé en ce que le capot (50) comporte une plaque frontale (52) tenue par des entretoises (53) à distance d'un anneau (51), qui glisse à frottement sur la périphérie du boîtier (20), avec une course suffisante pour que la plaque frontale (52) puisse venir obturer le fond antérieur du boîtier (20).

11. Nez artificiel selon la revendication 9, caractérisé en ce que le capot (60) comporte un bord latéral (63) qui s'étend jusqu'au fond antérieur (64) sur une demi-circonférence tandis que l'autre demi-circonférence débouche dans une jupe plate orientable (62).

12. Nez artificiel selon la revendication 8, caractérisé en ce qu'il comporte en outre, coiffant le boîtier (20) autour du fond antérieur, une chambre à clapets (72), avec un clapet d'aspiration (74) entre la chambre et une canalisation (73) apte à être plaquée sur le thorax du sujet en sorte d'être sensiblement à la température du corps, et un clapet d'expiration (75) débouchant directement sur l'extérieur.

## Claims

1. Artificial nose (1) for a tracheotomy subject with a surgical orifice (3) joining the trachea to the skin (2), having, in a perforated housing (20) held on the periphery of the surgical orifice (3) by an annular base (10) adhering to the skin, a hydrophilic filtering mass (40) forming an alternating-circulation heat and moisture exchanger between the exhaled air and the inhaled air, a grid (30) being disposed between the filtering mass (40) and the surgical orifice (3), characterised in that, the grid (30) being disposed in the housing with front (32) and rear (31) surfaces which are distant in their active central part and facing respectively towards the filtering mass (40) and the surgical orifice (3), the rear surface (31) has calibrated passages, and is spaced apart from the front surface (32) by a distance such, conjointly with the calibre of the passages, that mucus expelled from the trachea is not, for the main part, able to reach the filtering mass (40).

2. Artificial nose according to Claim 1, characterised in that the filtering mass (40) is produced from polyurethane foam with open pores.

3. Artificial nose according to one of Claims 1 and 2, characterised in that the base (10) has a flexible collar (11) surrounding a front annular rim (12) on which a complementary recess (21) in the housing (20) engages.

4. Artificial nose according to Claim 3, characterised in that the annular rim (12) has a peripheral ring (13) which engages in a groove (22) formed in the recess in the housing (20).

5. Artificial nose according to one of Claims 3 and 4, characterised in that the base (10) also has a rear sleeve (14) in line with the annular rim (12) and suitable for engaging in the surgical orifice.

6. Artificial nose according to any one of Claims 1 to 5, characterised in that the base (10) is provided with a double-sided adhesive (15), suitable for being applied to the skin (2).

7. Artificial nose according to any one of Claims 1 to 6, characterised in that the front surface (32) of the grid (30) is plane, whilst its rear surface (31) is curved in a dome shape, and consists of a grid with a square mesh.

8. Artificial nose according to any one of Claims 1 to 7, characterised in that the housing (20) is annular in shape with a lattice front end.

9. Artificial nose according to claim 8, characterised in that the housing (20) has a cap (50, 60, 70) covering the front end (52, 64) parallel to and at a distance from the latter, with lateral openings.

10. Artificial nose according to Claim 9, characterised in that the cap (50) has a front plate (52) held by struts (53) at a distance from an annulus (51), which slides with friction on the periphery of the housing (20), with sufficient travel for the front plate (52) to be able to came to close off the front end of the housing (20).

11. Artificial nose according to Claim 9, characterised in that the cap (60) has a lateral edge (63) which extends as far as the front end (64) over one semi-circumference whilst the other semi-circumference emerges in an orientable flat skirt (62).

12. Artificial nose according to Claim 8, characterised in that it also includes, fitting on top of the housing (20) around the front end, a chamber with valves (72), with an inspiration valve (74) between the chamber and a pipe (73) suitable for being pressed against the thorax of the subject so as to be substantially at the same temperature as the body, and an expiration valve (75) opening out directly to the outside.

## Patentansprüche

1. Künstliche Nase (1) für eine Person mit Luftröhrenschnitt mit einer chirurgischen Öffnung (3), die die Luftröhre mit der Haut (2) verbindet, welche künstliche Nase in einem durchbrochenen Gehäuse (20), das an der Peripherie der chirurgischen Öffnung (3) mittels einer ringförmigen an der Haut haftenden Sitzfläche (10) gehalten ist, eine hydrophile Filtriermasse (40) aufweist, die einen Austauscher zwischen der ausgeatmeten und eingeatmeten Luft mittels wechselnder Zirkulation von Wärme und Feuchtigkeit bildet, wobei ein Gitter (30) zwischen der Filtriermasse (40) und der chirurgischen Öffnung (3) angeordnet ist,
dadurch gekennzeichnet, daß das Gitter (30), das in dem Gehäuse mit einer Vorder- (32) und einer Rückseite (31) angeordnet ist, die in ihrem zentralen aktiven Teil beabstandet sind und jeweils zur Filtriermasse (40) oder der chirurgischen Öffnung (3) gerichtet sind, die Rückseite (31) kalibrierte Durchlässe besitzt, und zusammen mit der Bohrung bzw. dem Kaliber der Durchlässe derart von der Vorderseite beabstandet ist, daß der von der Luftröhre ausgeworfene Schleim im wesentlichen nicht die Filtriermasse (40) erreichen kann.

2. Künstliche Nase nach Anspruch 1,
dadurch gekennzeichnet, daß die Filtriermasse (40) in Polyurethan-Schaum mit offenen Poren ausgeführt ist.

3. Künstliche Nase nach Anspruch 1 oder 2,
dadurch gekennzeichnet, daß die Sitzfläche (10) einen elastischen Kragen (11) aufweist, der eine vordere ringförmige Leiste (12) umgibt, auf die eine zusätzliche Bohrung (21) des Gehäuses (20) mündet.

4. Künstliche Nase nach Anspruch 3,
dadurch gekennzeichnet, daß die ringförmige Leiste (12) einen peripheren Reifen (13) aufweist, der in eine in der Bohrung des Gehäuses (20) ausgeführten Kehle (22) eingreift.

5. Künstliche Nase nach Anspruch 3 oder 4,
dadurch gekennzeichnet, daß die Sitzfläche (10) ferner eine rückseitige Manschette (14) in der Verlängerung der ringförmigen Leiste (12) aufweist, die dazu geeignet ist, in die chirurgische Öffnung einzugreifen.

6. Künstliche Nase nach einem der Ansprüche 1 bis 5,
dadurch gekennzeichnet, daß die Sitzfläche (10) mit einem doppelseitigen Klebeband (15) versehen ist, das dazu geeignet ist, an der Haut (2) aufgetragen zu werden.

7. Künstliche Nase nach einem der Ansprüche 1 bis 6,
dadurch gekennzeichnet, daß die Vorderseite (32) des Gitters (30) eben ist, während seine Rückseite (31) schalenförmig gewölbt ist und aus einem Gitter mit viereckigen Maschen aufgebaut ist.

8. Künstliche Nase nach einem der Ansprüche 1 bis 7,
dadurch gekennzeichnet, daß das Gehäuse (20) eine ringförmige Form mit einem vorderen, mit einem Gitter versehenen Boden hat.

9. Künstliche Nase nach Anspruch 8,
dadurch gekennzeichnet, daß das Gehäuse (20) eine Haube (50, 60, 70) mit seitlichen Öffnungen aufweist, die den vorderen Boden (52, 64) parallel und von diesem beabstandet bedeckt.

10. Künstliche Nase nach Anspruch 9,
dadurch gekennzeichnet, daß die Haube (50) eine Frontplatte (52) aufweist, die mittels Stegen (53) von einem Ring (51) beabstandet gehalten ist, welche Haube unter Reibung auf der Peripherie des Gehäuses (20) mit ausreichendem Lauf gleitet, damit die Frontplatte (52) den vorderen Boden des Gehäuses (20) verschließen kann.

11. Künstliche Nase nach Anspruch 9,
dadurch gekennzeichnet, daß die Haube (60) einen seitlichen Rand (63) aufweist, der sich bis zum vorderen Boden (64) auf einem Halbkreis erstreckt, während der andere Halbkreis in eine einstellbare ebene Verkleidung (62) mündet.

12. Künstliche Nase nach Anspruch 8,
dadurch gekennzeichnet, daß sie ferner, wobei das Gehäuse (20) um den vorderen Boden herum überdeckt ist, eine Kammer mit Klappen (72) mit einer Einatmungsklappe (74) zwischen der Kammer und einer Leitung (73), welche geeignet ist, auf den Thorax der Person aufgelegt zu werden, so daß sie im wesentlichen der Temperatur des Körpers entspricht, und eine Ausatmungsklappe (75) aufweist, die direkt nach außen mündet.
